# EUROPÄISCHE PATENTSCHRIFT

(11) **EP 3 865 091 B1**
(45) Veröffentlichungstag und Bekanntmachung des Hinweises auf die Patenterteilung: **28.12.2022**
(21) Anmeldenummer: 21157023.9
(22) Anmeldetag: 15.02.2021
(51) Int. Cl.: A61F 2/00, A61F 2/38

(54) **VERPACKUNGSVORRICHTUNG FÜR EIN IMPLANTAT UND IMPLANTATANORDNUNG UMFASSEND EIN IMPLANTAT UND EINE VERPACKUNGSVORRICHTUNG**
PACKAGING DEVICE FOR AN IMPLANT AND IMPLANT ASSEMBLY COMPRISING AN IMPLANT AND A PACKAGING DEVICE
DISPOSITIF D'EMBALLAGE POUR UN IMPLANT ET DISPOSITIF D'IMPLANT COMPRENANT UN IMPLANT ET UN DISPOSITIF D'EMBALLAGE

(30) Priorität: 17.02.2020 DE 102020104087
(43) Veröffentlichungstag der Anmeldung: 18.08.2021
(73) Patentinhaber: Aesculap AG, 78532 Tuttlingen (DE)
(72) Erfinder: Kammerer, Selina, 78083 Dauchingen (DE)
(74) Vertreter: Hoeger, Stellrecht & Partner Patentanwälte mbB

(56) Entgegenhaltungen:
- WO-A1-2013/163160
- US-A- 5 148 920
- US-A- 5 772 031
- US-A1- 2013 277 261
- US-A1- 2018 036 092

## Beschreibung

Die vorliegende Erfindung betrifft eine Verpackungsvorrichtung für ein Implantat, insbesondere für ein eine Beschichtung aufweisendes Implantat, insbesondere für eine Tibiakomponente einer Kniegelenkprothese, welche Verpackungsvorrichtung ein erstes Verpackungsteil und ein zweites Verpackungsteil umfasst, die in einem Verpackungszustand einen Aufnahmeraum zwischen sich definieren, im Aufnahmeraum ein an einem der Verpackungsteile angeordnetes Aufnahmeelement, in dem ein insbesondere die Beschichtung aufweisender erster Implantatabschnitt aufnehmbar ist, und im Aufnahmeraum ein im Abstand zum Aufnahmeelement angeordnetes Stützelement, das an einen zweiten Implantatabschnitt anlegbar ist, wobei das Aufnahmeelement und/oder das Stützelement, zumindest abschnittsweise und bevorzugt vollständig, aus einem für die Beschichtung des Implantates abriebresistenten Material gefertigt sind oder eine abriebresistente Beschichtung aufweisen, und wobei das Aufnahmeelement und das Stützelement im Aufnahmeraum einander gegenüberliegend positioniert sind, zum Anlegen an zwei einander abgewandte Implantatabschnitte des Implantates.

Die Verpackungsvorrichtung ist insbesondere für eine Tibiakomponente einer Kniegelenkprothese vorgesehen.

Die vorliegende Erfindung betrifft ferner eine Implantatanordnung mit einem Implantat, insbesondere einer Tibiakomponente, und einer Verpackungsvorrichtung.

Medizintechnische Produkte wie insbesondere Implantate werden für den Transport und die Bevorratung in Verpackungsvorrichtungen verpackt. Infolge des Fertigungsprozesses oder Kontakt mit der Verpackung kann die Gefahr bestehen, dass das Implantat mit möglicherweise schadhaften Partikeln belastet ist. Herstellungstechnisch kann dies zum Beispiel daran liegen, dass selbst Reinräume nicht vollständig partikelfrei sind. Transportbedingt können sich beispielsweise Partikel der Verpackung lösen und das Implantat kontaminieren.

Es kann nicht ausgeschlossen werden, dass eine Kontaminierung des Implantates mit Partikeln postoperativ zu Beschwerden des Patienten führen kann und beispielsweise eine Lockerung des Implantates verursachen kann.

Die US 2008/0190805 A1 beschreibt eine Verpackungsvorrichtung für eine mit poröser Oberfläche versehene Tibiakomponente. Das Tibiaplateau ist in einem taschenartigen Aufnahmeelement der Verpackungsvorrichtung angeordnet und von diesem allseits umschlossen. Dies wirkt sich nicht nur nachteilig auf die Handhabung der Tibiakomponente aus. Beim Einsetzen in das Aufnahmeelement und beim Entnehmen aus dem Aufnahmeelement ist am Tibiaschaft anzugreifen. Es besteht das Risiko, dass der Schaft dabei kontaminiert wird.

Die US 2018/0036092 A1 beschreibt eine Blister-Verpackung für einen medizintechnischen Artikel. Dabei ist der Artikel beispielsweise in einer Innenverpackung aufgenommen, die ihrerseits in einer Außenverpackung angeordnet ist.

Eine weitere Implantatverpackung für eine Tibiakomponente einer Knieprothese ist in der CN 208326155 U beschrieben.

Aus der US 5,148,920 sind eine Verpackung und ein Verpackungseinsatz bekannt. Eine Verpackung für ein langgestrecktes medizinisches Produkt ist in der US 5,772,031 beschrieben. Eine Verpackung für einen Satz femoraler Komponenten unterschiedlicher Größe eines künstlichen Kniegelenks ist in der US 2013/0277261 A1 offenbart. Die WO 2013/163160 A1 betrifft Verpackungen für medizinische Artikel wie beispielsweise einen Satz orthopädischer Implantate unterschiedlicher Größe.

Aufgabe der vorliegenden Erfindung ist es, eine Verpackungsvorrichtung für ein Implantat und eine Implantatanordnung bereitzustellen, bei deren Einsatz eine höhere Patientensicherheit gewährleistet werden kann.

Diese Aufgabe wird erfindungsgemäß dadurch gelöst, dass die Verpackungsvorrichtung mindestens ein Vorspannelement umfasst, das das Stützelement mit einer auf das Aufnahmeelement gerichteten Vorspannkraft beaufschlagt, und dass zur Aufnahme des Implantats das Aufnahmeelement und das Stützelement entgegen der Vorspannkraft voneinander wegbewegt werden können.

Die erfindungsgemäße Verpackungsvorrichtung ist insbesondere vorgesehen für ein zumindest teilweise mit einer Beschichtung versehenes Implantat. Hierbei kann es sich insbesondere um eine Tibiakomponente einer Kniegelenkprothese handeln. Die Erfindung ist jedoch nicht hierauf beschränkt. Die Verpackungsvorrichtung kann auch mit andersartigen Implantaten zum Einsatz kommen. Bekannt sind beispielsweise poröse, osteointegrative Beschichtungen, die das Anwachsen des Knochens am Implantat fördern. Eine derartige Beschichtung weist typischerweise eine hohe Porosität auf, und es besteht eine Gefahr erhöhten Abriebs der Beschichtung mit der Verpackungsvorrichtung. Günstig ist es, dass die Verpackungsvorrichtung mindestens ein Vorspannelement umfasst, das das Stützelement mit einer auf das Aufnahmeelement gerichteten Vorspannkraft beaufschlagt. Dies bietet den Vorteil, das Implantat an den relativ zueinander vorgespannten Komponenten - Aufnahmeelement und Stützelement - zuverlässig zu halten. Vorteilhafterweise besteht der Vorteil, eine Mehrzahl von Implantaten unterschiedlicher Größe mit der Verpackungsvorrichtung zu verpacken, wobei die Vorspannkraft eine jeweilige Anpassung der Relativstellung beider Komponenten erlaubt. Bei kleineren Implantaten weisen das Aufnahmeelement und das Stützelement einen geringeren Abstand voneinander auf als bei größeren Implantaten, zu deren Aufnahme das Aufnahmeelement und das Stützelement entgegen der Vorspannkraft voneinander wegbewegt werden können.

Gemäß der vorliegenden Erfindung ist ein Aufnahmeelement an einem Verpackungsteil und ein Stützelement vorgesehen, die im Aufnahmeraum angeordnet sind. Ein erster Implantatabschnitt, der insbesondere die Beschichtung aufweist, kann im Aufnahmeteil aufgenommen werden. Ein zweiter Implantatabschnitt kann an das im Abstand dazu angeordnete Stützelement angelegt werden. Dies erlaubt es, das Implantat, insbesondere die Tibiakomponente, zuverlässig im Aufnahmeraum zu halten und den Kontakt mit den übrigen Komponenten der Verpackungsvorrichtung zu verringern und vorteilhaft zu eliminieren. Das Aufnahmeelement und/oder das Stützelement ist mit einem abriebresistenten Material beschichtet oder aus einem solchen gefertigt. Hierunter ist vorliegend insbesondere zu verstehen, dass die abriebresistente Beschichtung bei Kontakt mit dem Implantat und insbesondere dessen Beschichtung einer lediglich geringen Gefahr des Abriebs unterliegt und vorzugsweise ein Abrieb vollständig oder im Wesentlichen vollständig vermieden werden kann. Dies verringert die Gefahr einer Kontaminierung des Implantates und steigert die Patientensicherheit.

Es kann vorgesehen sein, dass nur das Aufnahmeelement oder nur das Stützelement die abriebresistente Beschichtung aufweist oder aus dieser gefertigt sind.

Vorgesehen ist, dass das Aufnahmeelement und das Stützelement im Aufnahmeraum einander gegenüberliegend positioniert sind, zum Anlegen an zwei einander abgewandte Implantatabschnitte des Implantates. Dabei ist das Implantat vorzugsweise zwischen dem Aufnahmeelement und dem Stützelement positioniert.

Das Aufnahmeelement und das Stützelement liegen einander im Aufnahmeraum beispielsweise unmittelbar gegenüber.

Das Aufnahmeelement und das Stützelement bilden vordefinierte Schnittstellen für die Implantatabschnitte. Im Fall einer Tibiakomponente kann insbesondere deren Schaft im Aufnahmeelement aufgenommen werden, und das Tibiaplateau kann am Stützelement angelegt werden.

Von Vorteil ist es, wenn das erste Verpackungsteil und das zweite Verpackungsteil frei von Kontakt mit dem Implantat sind und das Implantat, von den Komponenten der Verpackungseinrichtung, nur mit dem Aufnahmeelement und dem Stützelement in Kontakt steht. Ein Abrieb von Material der Verpackungsteile lässt sich dadurch vermeiden und die Patientensicherheit erhöhen.

Als vorteilhaft kann es sich erweisen, wenn die Verpackungsvorrichtung in sich rotationssymmetrisch oder im Wesentlichen rotationssymmetrisch bezüglich einer Symmetrieachse ausgebildet ist. Eine derartige Symmetrie kann beispielsweise vorgesehen sein, wenn, wie nachfolgend erläutert, ein Verpackungsteil zumindest näherungsweise die Gestalt einer Hohlkugelkalotte aufweist und das zweite Verpackungsteil beispielsweise eine plattenförmige Gestalt. Die Symmetrieachse kann beispielsweise quer und insbesondere senkrecht zu einer vom zweiten Verpackungsteil definierten Ebene ausgerichtet sein.

Beispielsweise kann vorgesehen sein, dass das Aufnahmeelement und das Stützelement axial, bezogen auf die vorstehend genannte Symmetrieachse, einander gegenüberliegend im Aufnahmeraum positioniert sind.

Das mindestens eine Vorspannelement kann zum Beispiel eine Druckfeder sein, insbesondere eine Schraubenfeder.

Die Vorspannkraft kann beispielsweise entlang einer Achse der Verpackungsvorrichtung, insbesondere der Symmetrieachse, und/oder entlang einer Achse des Implantates ausgerichtet sein. Beispielsweise ist das mindestens eine Vorspannelement entlang der Achse der Verpackungsvorrichtung und/oder entlang der Achse des Implantates ausgerichtet. Alternativ dazu kann mindestens ein Vorspannelement parallel zur Achse der Verpackungsvorrichtung und/oder parallel zur Achse des Implantates ausgerichtet sein. Die Achse des Implantates ist beispielsweise im Fall einer Tibiakomponente durch den Tibiaschaft definiert.

Die Achse der Verpackungsvorrichtung kann beispielsweise eine vom Aufnahmeelement definierte Achse sein. Vorgesehen sein kann, dass die Verpackungsvorrichtung eine Mehrzahl von Vorspannelementen umfasst.

Das mindestens eine Vorspannelement ist beispielsweise am zweiten Verpackungsteil festgelegt. Insbesondere kann das Vorspannelement zwischen dem zweiten Verpackungsteil und dem Stützelement angeordnet sein. Über das Vorspannelement kann das Stützelement mit dem zweiten Verpackungsteil verbunden sein.

Vorgesehen sein kann, dass das mindestens eine Vorspannelement am ersten Verpackungsteil festgelegt ist. Beispielsweise kann das Vorspannelement zwischen dem ersten Verpackungsteil und dem Aufnahmeelement angeordnet sein. Über das Vorspannelement kann das Aufnahmeelement mit dem ersten Verpackungsteil verbunden sein.

Bei einer andersartigen vorteilhaften Ausführungsform kann vorgesehen sein, dass das Aufnahmeelement unmittelbar am ersten Verpackungsteil festgelegt ist, ohne dazwischen angeordnetes Vorspannelement.

Vorgesehen sein kann, dass das Stützelement unmittelbar am zweiten Verpackungsteil festgelegt ist, ohne dazwischen angeordnetes Vorspannelement.

Vorgesehen sein kann, dass das Aufnahmeelement getrennt vom ersten Verpackungsteil gebildet und an diesem unmittelbar oder mittelbar festgelegt ist.

Alternativ kann das Aufnahmeelement vom ersten Verpackungsteil gebildet oder von diesem umfasst sein.

In entsprechender Weise kann es vorteilhaft sein, wenn das Stützelement getrennt vom zweiten Verpackungsteil gebildet und an diesem unmittelbar oder mittelbar festgelegt ist.

Alternativ kann das Stützelement vom zweiten Verpackungsteil gebildet oder von diesem umfasst sein.

Eine Festlegung oder Verbindung des Aufnahmeelementes und/oder des Stützelementes an bzw. mit einem jeweiligen Verpackungsteil kann auf alle möglichen Weisen erfolgen, insbesondere durch Formschluss, Kraftschluss und/oder Stoffschluss. Die Verbindung kann direkt oder indirekt sein. Vorgesehen sein kann bei einer bevorzugten Ausführungsform, dass das Aufnahmeteil mit Formschluss an das erste Verpackungsteil angelegt oder an dieses angespritzt ist. Das Stützelement kann beispielsweise über das vorstehend genannte Vorspannelement mit dem zweiten Verpackungsteil verbunden sein.

Die Fertigung des Aufnahmeelementes, des Stützelementes und/oder der Verpackungsteile kann auf unterschiedliche Weisen erfolgen. Die Verpackungsteile sind beispielsweise Spritzgussteile. Das Aufnahmeelement und/oder das Stützelement kann beispielsweise durch Spritzguss und/oder durch Tiefziehen hergestellt werden.

Das abriebresistente Material kann beispielsweise ein thermoplastisches Elastomer sein, insbesondere ein thermoplastisches Elastomer auf Urethanbasis (TPU). Andersartige geeignete thermoplastische Elastomere sind beispielsweise TPA (Thermoplastische Polyamidelastomere), TPC (Thermoplastische Copolyesterelastomere), TPO (Thermoplastische Elastomere auf Olefinbasis) oder TPV (Thermoplastische Vulkanisate oder vernetzte thermoplastische Elastomere auf Olefinbasis).

Bei einer bevorzugten Ausführungsform ist das Aufnahmeelement und/oder das Stützelement beispielsweise ein Tiefziehteil aus einer TPU-Folie oder ein Spritzgussteil aus TPU.

Das abriebresistente Material kann beispielsweise ein Polylactidmaterial (PLA) sein, im Hinblick auf eine Biokompatibilität des Materials.

Insbesondere kann vorzugsweise ein biokompatibles abriebrestistentes Material vorgesehen sein. Sollte Abrieb entstehen und sollten sich Partikel am Implantat anlagern, können diese im Körper resorbiert werden.

Günstig kann es sein, wenn das erste Verpackungsteil und/oder das zweite Verpackungsteil aus einem Material mit mindestens einer der folgenden Eigenschaften bestehen oder ein solches Material umfassen:
- gefertigt aus PP, PE und/oder PEEK;
- gefertigt aus einem beta- und/oder gammasterilisierbaren Material;
- gefertigt aus oder beschichtet mit einem abriebresistenten Material, beispielsweise einem der vorstehend genannten abriebresistenten Materialien.

Günstigerweise kann zum Beispiel vorgesehen sein, dass mindestens eines der Verpackungsteile dem Aufnahmeraum zugewandt eine abriebresistente Beschichtung aufweist. Die Gefahr eines Partikeleintrags auf das Implantat kann dadurch weiter reduziert werden.

Vorteilhaft kann es sein, wenn das Stützelement ein flächiges Anlageelement umfasst oder ausbildet zum flächigen Anliegen am zweiten Implantatabschnitt. Beispielsweise ist das Stützelement plattenförmig und geeignet zum flächigen Anliegen an einem flächigen Tibiaplateau einer Tibiakomponente.

Alternativ kann vorgesehen sein, dass das Stützelement eine Mehrzahl von diskreten, voneinander beabstandeten Stützgliedern zum jeweiligen Anlegen an den zweiten Implantatabschnitt umfasst. Dadurch kann die Kontaktfläche zwischen dem Implantatabschnitt und dem Stützelement verringert werden, um der Gefahr eines etwaigen Abriebs entgegenzuwirken.

Günstig kann es sein, wenn das Aufnahmeelement und/oder das Stützelement eine jeweilige Ausnehmung umfassen oder bilden, in die der erste Implantatabschnitt bzw. der zweite Implantatabschnitt eingreift und die einen Rand aufweist, der den ersten Implantatabschnitt bzw. den zweiten Implantatabschnitt bevorzugt vollständig umgibt. Beispielsweise kann ein jeweiliger Implantatabschnitt formschlüssig oder im Wesentlichen formschlüssig in das Aufnahmeelement bzw. das Stützelement eingreifen.

Das Aufnahmeelement kann zum Beispiel als Kappe oder Hülse ausgestaltet sein.

Das Stützelement kann zum Beispiel plattenförmig mit einem umlaufenden Rand ausgestaltet sein, wobei der Rand zum Beispiel in Richtung des Aufnahmeelementes absteht.

Günstig ist es, wenn das erste Verpackungsteil und das zweite Verpackungsteil lösbar miteinander verbindbar sind. Beispielsweise nehmen die Verpackungsteile einen Aufnahmezustand ein, indem sie voneinander gelöst sind und indem das Implantat am Aufnahmeelement oder am Stützelement angelegt wird. Ausgehend vom Aufnahmezustand können die Verpackungsteile miteinander verbunden und in den Verpackungszustand überführt werden.

Die Verpackungsteile können zum Beispiel mittels einer Schraubverbindung miteinander verbunden werden. Die Schraubverbindung kann insbesondere zum passgenauen Fixieren des Implantats zwischen dem Aufnahmeelement und dem Stützelement fixiert werden, wobei vorzugsweise entgegen der Wirkung des vorstehend genannten Vorspannelementes auf die Verpackungsteile eingewirkt wird.

Ein Betätigungselement an mindestens einem Verpackungsteil, zum Beispiel in Gestalt eines Griffelementes, kann zum Verschrauben vorgesehen sein.

Das erste und/oder das zweite Verpackungsteil können zumindest abschnittsweise aus einem transparenten Material gefertigt sein.

Vorgesehen sein kann, dass das erste Verpackungsteil, zumindest näherungsweise, die Gestalt einer Hohlkugelkalotte aufweist, speziell in Halbkugelform.

Das zweite Verpackungsteil kann beispielsweise eine im Wesentlichen plattenförmige Gestalt aufweisen zum Schließen des von der Hohlkugelkalotte überfangenen Aufnahmeraumes.

Das zweite Verpackungsteil kann beispielsweise als Deckel zum Verschließen des ersten Verpackungsteils ausgebildet sein. Der Deckel kann zum Beispiel eine Ebene definieren, quer und insbesondere senkrecht zu dem eine Achse der Verpackungseinrichtung definiert ist, insbesondere eine Symmetrieachse.

Wie eingangs erwähnt, betrifft die vorliegende Erfindung auch eine Implantatanordnung.

Eine die eingangs genannte Aufgabe lösende erfindungsgemäße Implantatanordnung umfasst ein Implantat mit einem ersten Implantatabschnitt und einem zweiten Implantatabschnitt sowie eine Verpackungsvorrichtung der vorstehend beschriebenen Art, wobei das Implantat in einem Verpackungszustand der Verpackungsvorrichtung im Aufnahmeraum aufgenommen ist, wobei der erste Implantatabschnitt im Aufnahmeelement aufgenommen ist und das Stützelement am zweiten Implantatabschnitt anliegt.

Die Vorteile, die bereits im Zusammenhang mit der Erläuterung der erfindungsgemäßen Verpackungsvorrichtung erwähnt wurden, können bei der Implantatanordnung ebenfalls erzielt werden. Diesbezüglich kann auf die voranstehenden Ausführungen verwiesen werden.

Vorteilhafte Ausführungsformen der erfindungsgemäßen Implantatanordnung ergeben sich durch vorteilhafte Ausführungsformen der erfindungsgemäßen Verpackungsvorrichtung. Diesbezüglich wird auf die voranstehenden Ausführungen verwiesen.

Das Implantat kann insbesondere eine Tibiakomponente einer Kniegelenkprothese sein, die einen Tibiaschaft und ein Tibiaplateau umfasst, wobei der Tibiaschaft den ersten Implantatabschnitt umfasst oder bildet und wobei das Tibiaplateau den zweiten Implantatabschnitt umfasst oder bildet.

Die nachfolgende Beschreibung einer bevorzugten Ausführungsform der Erfindung dient im Zusammenhang mit der Zeichnung der näheren Erläuterung der Erfindung. Es zeigen:
- Figur 1:: eine Implantatanordnung und eine Verpackungsvorrichtung gemäß der vorliegenden Erfindung zum Verpacken einer Tibiakomponente, wobei Verpackungsteile einen Verpackungszustand einnehmen, jeweils in bevorzugter Ausführungsform;
- Figur 2:: die Implantatanordnung aus Figur 1, wobei die Verpackungsteile einen Aufnahmezustand einnehmen;
- Figur 3:: eine Darstellung entsprechend Figur 2 bei jeweiligen zweiten bevorzugten Ausführungsformen der Erfindung; und
- Figur 4:: eine Darstellung entsprechend Figur 2 bei jeweiligen dritten bevorzugten Ausführungsformen der Erfindung.

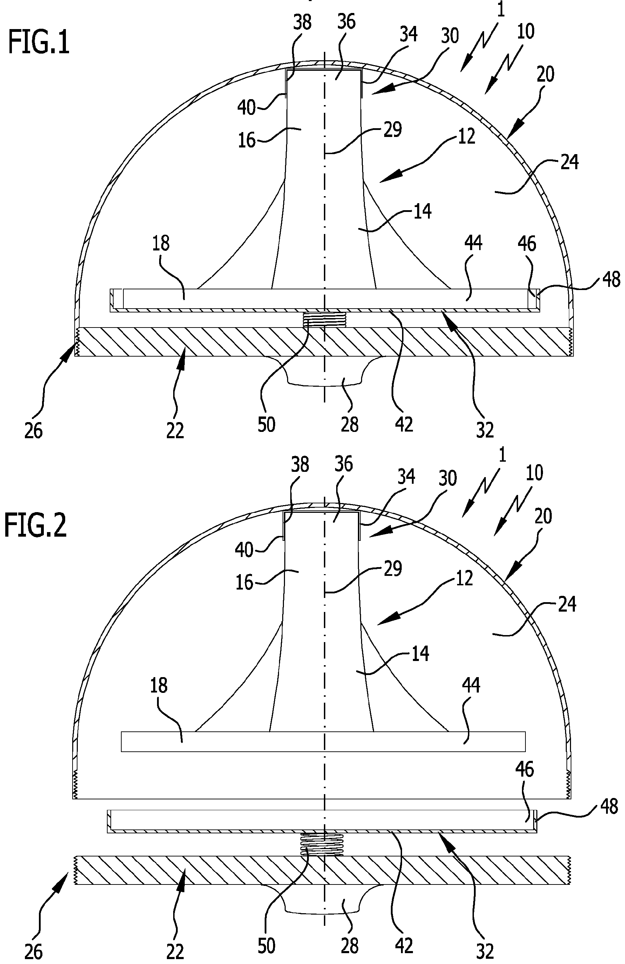

Die Figuren 1 und 2 zeigen eine insgesamt mit dem Bezugszeichen 1 belegte vorteilhafte Ausführungsform einer erfindungsgemäßen Implantatanordnung. Die Implantatanordnung 1 umfasst eine bevorzugte Ausführungsform einer erfindungsgemäßen Verpackungsvorrichtung sowie ein Implantat 12. Die Verpackungsvorrichtung ist insgesamt mit dem Bezugszeichen 10 gekennzeichnet. Bei dem Implantat 12 handelt es sich vorliegend um eine Tibiakomponente 14 einer Kniegelenkprothese. Die Tibiakomponente 14 weist in an sich bekannter Weise einen Schaft 16 und ein Tibiaplateau 18 auf.

Im vorliegenden Fall ist insbesondere vorgesehen, dass die Tibiakomponente 14 zumindest abschnittsweise eine Beschichtung aufweist. Die Beschichtung ist außenseitig aufgebracht und in der Zeichnung nicht dargestellt. Insbesondere kann der Schaft 16 die Beschichtung aufweisen.

Die Beschichtung kann insbesondere osteointegrativ sein, um das Anwachsen von Knochen an der Tibiakomponente 14 zu begünstigen. Aufgrund dessen weist das Implantat 12 im Bereich der Beschichtung eine hohe Rauheit auf.

Am Tibiaplateau 18 ist die Tibiakomponente 14 vorliegend frei von einer Beschichtung.

Die Verpackungsvorrichtung 10 umfasst ein erstes Verpackungsteil 20 und ein zweites Verpackungsteil 22, die in einem Verpackungszustand (Figur 1) einen Aufnahmeraum 24 für das Implantat 12 zwischen sich definieren und begrenzen.

Die Verpackungsteile 20, 22 sind lösbar miteinander verbindbar und können ausgehend von einem Aufnahmezustand (Figur 2), in dem sie voneinander getrennt sind, miteinander verbunden und in den Verpackungszustand überführt werden. Ausgehend vom Verpackungszustand können die Verpackungsteile 20, 22 voneinander gelöst und wieder in den Aufnahmezustand überführt werden.

Das erste Verpackungsteil 20 ist im vorliegenden Fall hohlkugelkalottenförmig gebildet und beispielsweise als Spritzgussteil gefertigt, zum Beispiel aus einer PETG-Folie. Vorzugsweise kann das erste Verpackungsteil innenseitig, dem Aufnahmeraum 24 zugewandt, zumindest abschnittsweise und bevorzugt vollständig mit einem abriebresistenten Material beschichtet sein. Das abriebresistente Material ist vorzugsweise ein thermoplastisches Material, insbesondere TPU.

Das erste Verpackungsteil 20 weist die Gestalt einer Halbkugel auf. Das zweite Verpackungsteil 20 ist im Wesentlichen von plattenförmiger Gestalt. Das zweite Verpackungsteil 22 bildet einen Deckel für das erste Verpackungsteil 20 und kann über eine Verbindungseinrichtung 26 mit dem ersten Verpackungsteil 20 verbunden werden. Vorliegend ist die Verbindungseinrichtung 26 eine Schraubverbindung, wobei das erste Verpackungsteil 20 ein Innengewinde und das zweite Verpackungsteil 22 ein Außengewinde aufweist.

Am zweiten Verpackungsteil 22 ist ein Betätigungselement 28 vorgesehen, ausgestaltet als Griffelement. Am Betätigungselement 28 kann das Verpackungsteil 22 zum Verschrauben mit dem Verpackungsteil 20 oder zum Lösen der Schraubverbindung ergriffen werden.

Die Verpackungsvorrichtung 10 ist insgesamt, abgesehen vom Betätigungselement 28, in sich rotationssymmetrisch bezüglich einer Symmetrieachse 29. Die Symmetrieachse 29 ist vorliegend quer und insbesondere senkrecht zu einer vom Verpackungsteil 22 definierten Ebene ausgerichtet. Die Symmetrieachse 29 verläuft durch den Pol der vom ersten Verpackungsteil 20 gebildeten Hohlkugelkalotte.

Die Verpackungsvorrichtung 10 umfasst ein Aufnahmeelement 30 und ein Stützelement 32. Das Aufnahmeelement 30 ist vorliegend in Form einer Kappe 34 gebildet. Die Kappe 34 ist gesondert vom ersten Verpackungsteil 20 gebildet und an diesem festgelegt, zum Beispiel durch Kraftschluss, Formschluss und/oder Stoffschluss.

Das Aufnahmeelement 30 definiert eine Achse, die vorliegend mit der Symmetrieachse 29 zusammenfällt.

Das Aufnahmeelement 30 ist aus einem abriebresistenten Material gefertigt, insbesondere einem thermoplastischen Elastomer, speziell aus TPU. Beispielsweise ist das Aufnahmeelement 30 durch Tiefziehen hergestellt.

Das Aufnahmeelement 30 dient zum Aufnehmen eines die osteointegrative Beschichtung aufweisenden ersten Implantatabschnittes 36 der Tibiakomponente 14. Der erste Implantatabschnitt 36 ist ein dem Tibiaplateau 18 abgewandter Abschnitt des Schaftes 16.

Das Aufnahmeelement 30 bildet eine Ausnehmung 38, in der der erste Implantatabschnitt 36 eingreift. Darüber hinaus umfasst das Aufnahmeelement 30 einen den ersten Implantatabschnitt 36 umgebenden Rand 40. Vorzugsweise übergreift das Aufnahmeelement 30 den Implantatabschnitt 36 formschlüssig.

Eine vom Schaft 16 definierte Achse fällt vorliegend mit der Achse des Aufnahmeelementes 30 und damit mit der Symmetrieachse 29 zusammen.

Durch die Fertigung des Aufnahmeelementes 30 aus einem abriebresistenten Material ist die Gefahr verringert, dass Partikel infolge des Kontaktes mit der Beschichtung des Schaftes 16 abgelöst werden und zu einer Kontamination des Implantates 12 führen.

Das Stützelement 32 ist im Wesentlichen plattenförmig ausgestaltet und im vorliegenden Fall von der Gestalt eines Tellers 42. Die Tibiakomponente 14 kann mit dem einen zweiten Implantatabschnitt 44 bildenden Tibiaplateau 18 am Stützelement 32 anliegen. Das Stützelement 32 bildet hierfür ein Anlageelement. Insbesondere umfasst das Stützelement 32 eine Ausnehmung 46, in die das Tibiaplateau 18 eingreifen kann. Ein Rand 48 umgibt den zweiten Implantatabschnitt 44.

Vorliegend weist das Stützelement 32 keine gesonderte abriebresistente Beschichtung auf. Dies kann jedoch im Rahmen der vorliegenden Erfindung selbstständig vorgesehen sein. Auch eine vollständige Fertigung aus abriebresistentem Material ist denkbar.

Das Aufnahmeelement 30 und das Stützelement 32 liegen einander im Aufnahmeraum 24 gegenüber und sind dadurch voneinander beabstandet. Das Implantat 12 ist zwischen dem Aufnahmeelement 30 und dem Stützelement 32 angeordnet.

Im vorliegenden Fall liegen das Aufnahmeelement 30 und das Stützelement einander axial, bezogen auf die Symmetrieachse 29, gegenüber. Insbesondere liegen das Aufnahmeelement 30 und das Stützelement 32 einander unmittelbar gegenüber, wobei sie jeweils koaxial zur Symmetrieachse 29 ausgerichtet sind.

Das Implantat 12 ist insbesondere frei von Kontakt mit den Verpackungsteilen 20 und 22 und steht nur mit dem Aufnahmeelement 30 und dem Stützelement 32 in Kontakt.

Das Stützelement 32 ist über ein Vorspannelement 50 am zweiten Verpackungsteil 22 festgelegt. Das Vorspannelement 50 ist als Druckfeder ausgestaltet, insbesondere als Schraubenfeder. Das Vorspannelement 50 beaufschlagt das Stützelement 32 mit einer auf das Aufnahmeelement 30 gerichteten Kraft, wodurch das Implantat 12 zuverlässig zwischen dem Aufnahmeelement 30 und dem Stützelement 32 fixiert ist.

Die Vorspannkraft des Vorspannelementes 50 ist vorliegend entlang der Symmetrieachse 29 ausgerichtet. Das Vorspannelement 50 ist koaxial zur Symmetrieachse 29 ausgerichtet.

Vorgesehen sein kann, dass mehr als nur ein Vorspannelement 50 zum Einsatz kommt. Beispielsweise sind zwei oder mehr Vorspannelemente vorgesehen, die vorzugsweise symmetrisch relativ zueinander bezüglich der Symmetrieachse 29 angeordnet sein können.

Darüber hinaus besteht die Möglichkeit, Implantate 12 unterschiedlicher Größe in der Verpackungsvorrichtung 10 zu fixieren. Die jeweilige Anpassung an die Größe erfolgt unter der Wirkung des Vorspannelementes 50.

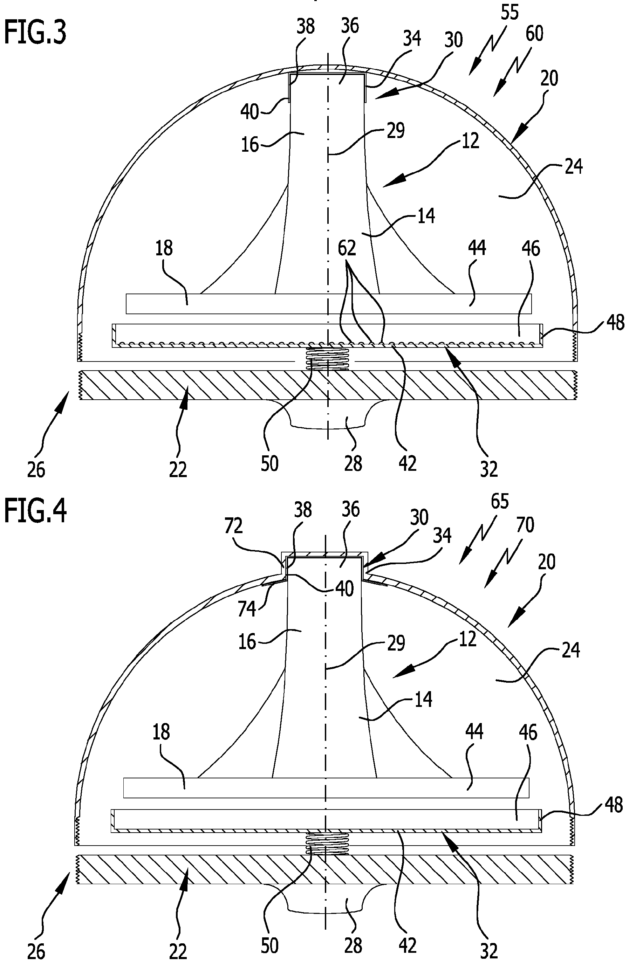

Nachfolgend wird auf in den Figuren 3 und 4 in einer der Figur 2 entsprechenden Weise dargestellte bevorzugte Ausführungsformen der erfindungsgemäßen Implantatanordnung und der erfindungsgemäßen Verpackungsvorrichtung eingegangen. Die im Zusammenhang mit der Implantatanordnung 1 und der Verpackungsvorrichtung 10 erwähnten Vorteile können mit diesen Verpackungsvorrichtungen ebenfalls erzielt werden, so dass diesbezüglich auf die voranstehenden Ausführungen verwiesen wird. Es werden lediglich die wesentlichsten Unterschiede erläutert.

Bei einer Implantatanordnung 55 mit einer Verpackungsvorrichtung 60 gemäß Figur 3 umfasst das Stützelement 32 einzelne, voneinander beabstandete diskrete Stützglieder 62. Die Stützglieder 62 verringern die Kontaktfläche des Stützelementes 32 mit dem zweiten Implantatabschnitt 44. Die Gefahr eines etwaigen Partikeleintrags am Implantat 12 wird dadurch verringert.

Die Implantatanordnung 65 mit der Verpackungsvorrichtung 70 gemäß Figur 4 sieht abweichend von der Verpackungsvorrichtung 10 vor, dass am ersten Verpackungsteil 20 eine Aufnahme 72 für das Aufnahmeelement 30 vorhanden ist. In der Aufnahme 72 weicht das Verpackungsteil 20 von der ansonsten hohlkugelkalottenförmigen Gestalt ab. Das Aufnahmeelement 30 steht in formschlüssigem Eingriff mit der Aufnahme 72. Auf diese Weise kann eine besonders zuverlässige Fixierung des Aufnahmeelementes 30 am ersten Verpackungsteil 20 erzielt werden.

Das Aufnahmeteil 30 umfasst ferner einen vom Rand 40 abstehenden Anlagebereich 74, der flanschartig am Verpackungsteil 20 anliegt und mit diesem zum Beispiel durch Kraftschluss oder Stoffschluss verbunden sein kann.

Es versteht sich, dass bei der Verpackungsvorrichtung 70 ebenfalls Stützglieder 62 am Stützelement 32 vorgesehen sein können.

### Bezugszeichenliste:

- 1: Implantatanordnung
- 10: Verpackungsvorrichtung
- 12: Implantat
- 14: Tibiakomponente
- 16: Schaft
- 18: Tibiaplateau
- 20: erstes Verpackungsteil
- 22: zweites Verpackungsteil
- 24: Aufnahmeraum
- 26: Verbindungseinrichtung
- 28: Betätigungselement
- 29: Symmetrieachse
- 30: Aufnahmeelement
- 32: Stützelement
- 34: Kappe
- 36: erster Implantatabschnitt
- 38: Ausnehmung
- 40: Rand
- 42: Teller
- 44: zweiter Implantatabschnitt
- 46: Ausnehmung
- 48: Rand
- 50: Vorspannelement
- 55: Implantatanordnung
- 60: Verpackungsvorrichtung
- 62: Stützglied
- 65: Implantatanordnung
- 70: Verpackungsvorrichtung
- 72: Aufnahme
- 74: Anlagebereich

## Patentansprüche

1. Verpackungsvorrichtung für ein eine Beschichtung aufweisendes Implantat (12), insbesondere für eine Tibiakomponente (14) einer Kniegelenkprothese, welche Verpackungsvorrichtung (10; 60; 70) ein erstes Verpackungsteil (20) und ein zweites Verpackungsteil (22) umfasst, die in einem Verpackungszustand einen Aufnahmeraum (24) zwischen sich definieren, im Aufnahmeraum (24) ein an einem der Verpackungsteile (20, 22) angeordnetes Aufnahmeelement (30), in dem ein insbesondere die Beschichtung aufweisender erster Implantatabschnitt (36) aufnehmbar ist, und im Aufnahmeraum (24) ein im Abstand zum Aufnahmeelement (30) angeordnetes Stützelement (32), das an einen zweiten Implantatabschnitt (44) anlegbar ist, wobei das Aufnahmeelement (30) und/oder das Stützelement (32), zumindest abschnittsweise und bevorzugt vollständig, aus einem für die Beschichtung des Implantates (12) abriebresistenten Material gefertigt sind oder eine abriebresistente Beschichtung aufweisen, und wobei das Aufnahmeelement (30) und das Stützelement (32) im Aufnahmeraum (24) einander gegenüberliegend positioniert sind, zum Anlegen an zwei einander abgewandte Implantatabschnitte (36, 44) des Implantates, **dadurch gekennzeichnet, dass** die Verpackungsvorrichtung (10; 60; 70) mindestens ein Vorspannelement (50) umfasst, das das Stützelement (32) mit einer auf das Aufnahmeelement (30) gerichteten Vorspannkraft beaufschlagt, und dass zur Aufnahme des Implantats (12) das Aufnahmeelement (30) und das Stützelement (32) entgegen der Vorspannkraft voneinander wegbewegt werden können.

2. Verpackungsvorrichtung nach Anspruch 1, **dadurch gekennzeichnet, dass** das erste Verpackungsteil (20) und das zweite Verpackungsteil (22) frei von Kontakt mit dem Implantat (12) sind und das Implantat (12), von den Komponenten der Verpackungsvorrichtung (10; 60; 70), nur mit dem Aufnahmeelement (30) und dem Stützelement (32) in Kontakt steht.

3. Verpackungsvorrichtung nach einem der voranstehenden Ansprüche, **dadurch gekennzeichnet, dass** die Verpackungsvorrichtung (10; 60; 70) in sich rotationssymmetrisch oder im Wesentlichen rotationssymmetrisch bezüglich einer Symmetrieachse (29) ausgebildet ist.

4. Verpackungsvorrichtung nach Anspruch 3, **dadurch gekennzeichnet, dass** das Aufnahmeelement (30) und das Stützelement (32) axial, bezogen auf die Symmetrieachse (29) einander gegenüberliegend im Aufnahmeraum (24) positioniert sind.

5. Verpackungsvorrichtung nach einem der voranstehenden Ansprüche, **dadurch gekennzeichnet, dass** das mindestens eine Vorspannelement (50) vorzugsweise als Druckfeder ausgestaltet ist, insbesondere als Schraubenfeder.

6. Verpackungsvorrichtung nach Anspruch 5, **dadurch gekennzeichnet, dass** die Vorspannkraft entlang einer von der Verpackungsvorrichtung (10; 60; 70) definierten Achse, insbesondere einer Symmetrieachse (29), und/oder entlang einer Achse des Implantates (12) ausgerichtet ist.

7. Verpackungsvorrichtung nach einem der voranstehenden Ansprüche, **dadurch gekennzeichnet, dass** zumindest eines der Folgenden gilt:
- das Aufnahmeelement (30) ist getrennt vom ersten Verpackungsteil (20) gebildet und an diesem festgelegt, und/oder das Stützelement (32) ist getrennt vom zweiten Verpackungsteil (22) gebildet und an diesem festgelegt;
- das Aufnahmeelement (30) ist vom ersten Verpackungsteil (20) gebildet oder von diesem umfasst und/oder das Stützelement (32) ist vom zweiten Verpackungsteil (22) gebildet oder von diesem umfasst.

8. Verpackungsvorrichtung nach einem der voranstehenden Ansprüche, **dadurch gekennzeichnet, dass** das abriebresistente Material eines der Folgenden ist oder umfasst:
- ein thermoplastisches Elastomer, insbesondere ein thermoplastisches Elastomer auf Urethanbasis (TPU);
- ein Polylactidmaterial (PLA);
- ein biokompatibles Material.

9. Verpackungsvorrichtung nach einem der voranstehenden Ansprüche, **dadurch gekennzeichnet, dass** das erste Verpackungsteil (20) und/oder das zweite Verpackungsteil (22) aus einem Material mit mindestens einer der folgenden Eigenschaften bestehen oder ein solches Material umfassen:
- gefertigt aus PP, PE und/oder PEEK;
- gefertigt aus einem beta- und/oder gammasterilisierbaren Material;
- gefertigt aus oder beschichtet mit einem abriebresistenten Material.

10. Verpackungsvorrichtung nach einem der voranstehenden Ansprüche, **dadurch gekennzeichnet, dass** das Stützelement (32) ein flächiges Anlageelement umfasst oder ausbildet zum flächigen Anliegen am zweiten Implantatabschnitt (44) oder dass das Stützelement (32) eine Mehrzahl von diskreten, voneinander beabstandeten Stützgliedern (62) zum jeweiligen Anlegen an den zweiten Implantatabschnitt (44) umfasst.

11. Verpackungsvorrichtung nach einem der voranstehenden Ansprüche, **dadurch gekennzeichnet, dass** das Aufnahmeelement (30) und/oder das Stützelement (32) eine jeweilige Ausnehmung (38, 46) umfassen oder bilden, in die der erste Implantatabschnitt (36) bzw. der zweite Implantatabschnitt (44) eingreift und die einen Rand (40, 48) aufweist, der den ersten Implantatabschnitt (36) bzw. den zweiten Implantatabschnitt (44) bevorzugt vollständig umgibt.

12. Verpackungsvorrichtung nach einem der voranstehenden Ansprüche, **dadurch gekennzeichnet, dass** das erste Verpackungsteil (20) und das zweite Verpackungsteil (22) lösbar miteinander verbindbar sind, vorzugsweise mittels einer Schraubverbindung, wobei insbesondere ein Betätigungselement (28) an mindestens einem der Verpackungsteile (20, 22) angeordnet ist.

13. Verpackungsvorrichtung nach einem der voranstehenden Ansprüche, **dadurch gekennzeichnet, dass** zumindest eines der Folgenden gilt:
- das erste Verpackungsteil (20) und/oder das zweite Verpackungsteil (22) ist zumindest abschnittsweise aus einem transparenten Material gefertigt;
- das erste Verpackungsteil (20) weist, zumindest näherungsweise, die Gestalt einer Hohlkugelkalotte auf, und das zweite Verpackungsteil (22) weist eine im Wesentlichen plattenförmige Gestalt auf zum Schließen des von der Hohlkugelkalotte überfangenen Aufnahmeraumes (24).

14. Implantatanordnung, umfassend ein Implantat (12) mit einem ersten Implantatabschnitt (36) und einem zweiten Implantatabschnitt (44) sowie eine Verpackungsvorrichtung (10;60; 70) nach einem der voranstehenden Ansprüche, wobei das Implantat (12) in einem Verpackungszustand der Verpackungsvorrichtung (10; 60; 70) im Aufnahmeraum (24) aufgenommen ist, wobei der erste Implantatabschnitt (36) im Aufnahmeelement (30) aufgenommen ist und das Stützelement (32) am zweiten Implantatabschnitt (44) anliegt.

15. Implantatanordnung nach Anspruch 14, **dadurch gekennzeichnet, dass** das Implantat (12) eine Tibiakomponente (14) einer Kniegelenkprothese ist, die einen Tibiaschaft (16) und ein Tibiaplateau (18) umfasst, wobei der Tibiaschaft (16) den ersten Implantatabschnitt (36) umfasst oder bildet, und wobei das Tibiaplateau (18) den zweiten Implantatabschnitt (44) umfasst oder bildet.

## Claims

1. Packaging device for an implant (12) comprising a coating, in particular for a tibial component (14) of a knee joint prosthesis, which packaging device (10; 60; 70) comprises a first packaging part (20) and a second packaging part (22), which in a packaging state define between them a receiving space (24), in the receiving space (24) a receiving element (30) arranged on one of the packaging parts (20, 22), in which receiving element (30) a first implant portion (36), in particular comprising the coating, can be accommodated, and in the receiving space (24) a support element (32) arranged at a distance from the receiving element (30), which support element (32) can abut against a second implant portion (44), wherein the receiving element (30) and/or the support element (30) are made, at least in sections and preferably completely, of a material that is abrasion resistant for the coating of the implant (12) or comprise an abrasion resistant coating, and wherein the receiving element (30) and the support element (32) are positioned opposite one another in the receiving space (24), for being placed against two averted implant portions (36, 44) of the implant, **characterized in that** the packaging device (10; 60; 70) comprises at least one biasing element (50) that acts upon the support element (32) with a biasing force directed at the receiving element (30), and **in that** the receiving element (30) and the support element (32) can be moved away from one another against the biasing force for accommodating the implant (12).

2. Packaging device in accordance with Claim 1, **characterized in that** the first packaging part (20) and the second packaging part (22) are free of contact with the implant (12) and the implant (12), of the components of the packaging device (10; 60; 70), is only in contact with the receiving element (30) and the support element (32).

3. Packaging device in accordance with any one of the preceding Claims, **characterized in that** the packaging device (10; 60; 70) is of rotationally symmetrical or substantially rotationally symmetrical configuration with respect to an axis of symmetry (29).

4. Packaging device in accordance with Claim 3, **characterized in that** the receiving element (30) and the support element (32) are positioned axially opposite one another in the receiving space (24) with respect to the axis of symmetry (29).

5. Packaging device in accordance with any one of the preceding Claims, **characterized in that** the at least one biasing element (50) is preferably configured as a compression spring, in particular as a coil spring.

6. Packaging device in accordance with Claim 5, **characterized in that** the biasing force is directed along an axis defined by the packaging device (10; 60; 70), in particular an axis of symmetry (29), and/or along an axis of the implant (12).

7. Packaging device in accordance with any one of the preceding Claims, **characterized in that** at least one of the following applies:
- the receiving element (30) is formed separately from the first packaging part (20) and is fixed thereto, and/or the support element (32) is formed separately from the second packaging part (22) and is fixed thereto;
- the receiving element (30) is formed by the first packaging part (20) or is comprised thereby and/or the support element (32) is formed by the second packaging part (22) or is comprised thereby.

8. Packaging device in accordance with any one of the preceding Claims, **characterized in that** the abrasion resistant material is or comprises one of the following:
- a thermoplastic elastomer, in particular a thermoplastic elastomer based on urethane (TPU);
- a polylactide material (PLA);
- a biocompatible material.

9. Packaging device in accordance with any one of the preceding Claims, **characterized in that** the first packaging part (20) and/or the second packaging part (22) consist of or comprise a material with at least one of the following properties:
- made of PP, PE, and/or PEEK;
- made of a beta- and/or gamma-sterilizable material;
- made of or coated with an abrasion resistant material.

10. Packaging device in accordance with any one of the preceding Claims, **characterized in that** the support element (32) comprises or forms a flat abutment element for abutting in surface-to-surface contact against the second implant portion (44) or **in that** the support element (32) comprises a plurality of discrete support members (62) spaced at a distance from one another for each being placed against the second implant portion (44).

11. Packaging device in accordance with any one of the preceding Claims, **characterized in that** the receiving element (30) and/or the support element (32) each comprise or form a recess (38, 46) into which the first implant portion (36) or the second implant region (44) engages, and which each has a rim (40, 48) that preferably completely surrounds the first implant portion (36) or the second implant portion (44).

12. Packaging device in accordance with any one of the preceding Claims, **characterized in that** the first packaging part (20) and the second packaging part (22) are releasably connectable to one another, preferably by means of a screw connection, wherein, in particular, an actuating element (28) is arranged on at least one of the packaging parts (20, 22).

13. Packaging device in accordance with any one of the preceding Claims, **characterized in that** at least one of the following applies:
- the first packaging part (20) and/or the second packaging part (22) is made of a transparent material at least in sections;
- the first packaging part (20) has, at least approximately, the form of a hollow spherical calotte, and the second packaging part (22) has a substantially plate-shaped form for closing the receiving space (24) delimited by the hollow spherical calotte.

14. Implant arrangement, comprising an implant (12) with a first implant portion (36) and a second implant portion (44) as well as a packaging device (10; 60; 70) in accordance with any one of the preceding Claims, wherein the implant (12) in a packaging state of the packaging device (10; 60; 70) is accommodated in the receiving space (24), wherein the first implant portion (36) is accommodated in the receiving element (30) and the support element (32) abuts against the second implant portion (44).

15. Implant arrangement in accordance with Claim 14, **characterized in that** the implant (12) is a tibial component (14) of a knee joint prosthesis, which comprises a tibial shaft (16) and a tibial plateau (18), wherein the tibial shaft (16) comprises or forms the first implant portion (36), and wherein the tibial plateau (18) comprises or forms the second implant portion (44).

## Revendications

1. Dispositif de conditionnement pour un implant (12) comportant un revêtement, en particulier pour un composant tibial (14) d'une prothèse d'articulation du genou, lequel dispositif de conditionnement (10; 60; 70) comprend une première partie de conditionnement (20) et une deuxième partie de conditionnement (22), qui définissent entre elles un espace de réception (24) dans un état de conditionnement, dans l'espace de réception (24) un élément de réception (30) disposé sur l'une des parties de conditionnement (20, 22), dans lequel une première section d'implant (36) comportant en particulier le revêtement peut être reçue, et dans l'espace de réception (24) un élément de soutien (32) disposé à distance de l'élément de réception (30), qui peut être appliqué sur une deuxième section d'implant (44), où l'élément de réception (30) et/ou l'élément de soutien (32) sont produits, au moins par sections et de préférence complètement, en un matériau résistant à l'abrasion pour le revêtement de l'implant (12) ou comportent un revêtement résistant à l'abrasion, et où l'élément de réception (30) et l'élément de soutien (32) sont positionnés l'un en face de l'autre dans l'espace de réception (24), pour l'application sur deux sections d'implant (36, 44) opposées l'une à l'autre de l'implant, **caractérisé en ce que** le dispositif de conditionnement (10; 60; 70) comporte au moins un élément de précontrainte (50) qui sollicite l'élément de soutien (32) avec une force de précontrainte dirigée vers l'élément de réception (30), et **en ce que** pour la réception de l'implant (12), l'élément de réception (30) et l'élément de soutien (32) peuvent être éloignés l'un de l'autre contre la force de précontrainte.

2. Dispositif de conditionnement selon la revendication 1, **caractérisé en ce que** la première partie de conditionnement (20) et la deuxième partie de conditionnement (22) sont sans contact avec l'implant (12) et l'implant (12), parmi les composants du dispositif de conditionnement (10; 60; 70), n'est en contact qu'avec l'élément de réception (30) et l'élément de soutien (32).

3. Dispositif de conditionnement selon l'une des revendications précédentes, **caractérisé en ce que** le dispositif de conditionnement (10; 60; 70) est conçu intrinsèquement de manière symétrique en rotation ou sensiblement de manière symétrique en rotation par rapport à un axe de symétrie (29).

4. Dispositif de conditionnement selon la revendication 3, **caractérisé en ce que** l'élément de réception (30) et l'élément de soutien (32) sont positionnés axialement l'un en face de l'autre, par rapport à l'axe de symétrie (29) dans l'espace de réception (24).

5. Dispositif de conditionnement selon l'une des revendications précédentes, **caractérisé en ce que** le au moins un élément de précontrainte (50) est de préférence conçu comme un ressort de compression, en particulier comme un ressort hélicoïdal.

6. Dispositif de conditionnement selon la revendication 5, **caractérisé en ce que** la force de précontrainte est orientée le long d'un axe défini par le dispositif de conditionnement (10; 60; 70), en particulier d'un axe de symétrie (29), et/ou le long d'un axe de l'implant (12).

7. Dispositif de conditionnement selon l'une des revendications précédentes, **caractérisé en ce qu'**au moins l'un des éléments suivants s'applique:
- l'élément de réception (30) est formé séparément de la première partie de conditionnement (20) et fixé à celle-ci, et/ou l'élément de soutien (32) est formé séparément de la deuxième partie de conditionnement (22) et fixé à celle-ci;
- l'élément de réception (30) est formé par la première partie de conditionnement (20) ou est compris par celle-ci et/ou l'élément de soutien (32) est formé par la deuxième partie de conditionnement (22) ou est compris par celle-ci.

8. Dispositif de conditionnement selon l'une des revendications précédentes, **caractérisé en ce que** le matériau résistant à l'abrasion est ou comprend l'un des suivants:
- Un élastomère thermoplastique, en particulier un élastomère thermoplastique à base d'uréthane (TPU);
- un matériau polylactide (PLA);
un matériau biocompatible.

9. Dispositif de conditionnement selon l'une des revendications précédentes, **caractérisé en ce que** la première partie de conditionnement (20) et/ou la deuxième partie de conditionnement (22) consistent en un matériau avec au moins l'une des propriétés suivantes ou comprennent un tel matériau:
- produites en PP, PE et/ou PEEK;
- produites en un matériau stérilisable aux rayons bêta et/ou gamma;
- produites en ou recouvertes d'un matériau résistant à l'abrasion.

10. Dispositif de conditionnement selon l'une des revendications précédentes, **caractérisé en ce que** l'élément de soutien (32) comprend un ou est formé d'un élément d'application plan pour l'application à plat sur la deuxième section d'implant (44) ou **en ce que** l'élément de soutien (32) comprend une pluralité de membres de soutien (62) discrets et distants les uns des autres pour l'application respective sur la deuxième section d'implant (44).

11. Dispositif de conditionnement selon l'une des revendications précédentes, **caractérisé en ce que** l'élément de réception (30) et/ou l'élément de soutien (32) comprennent ou forment un évidement respectif (38, 46), dans lequel la première section d'implant (36) ou la deuxième section d'implant (44) pénètre et qui comporte un bord (40, 48) qui entoure de préférence totalement la première section d'implant (36) ou la deuxième section d'implant (44).

12. Dispositif de conditionnement selon l'une des revendications précédentes, **caractérisé en ce que** la première partie de conditionnement (20) et la deuxième partie de conditionnement (22) peuvent être reliées l'une à l'autre de manière amovible, de préférence au moyen d'une liaison à vis, où en particulier un élément d'actionnement (28) est disposé sur au moins l'une des parties de conditionnement (20, 22).

13. Dispositif de conditionnement selon l'une des revendications précédentes, **caractérisé en ce qu'**au moins l'un des éléments suivants s'applique:
- la première partie de conditionnement (20) et/ou la deuxième partie de conditionnement (22) est produite au moins par sections en un matériau transparent;
- la première partie de conditionnement (20) présente, au moins approximativement, la forme d'une calotte sphérique creuse, et la deuxième partie de conditionnement (22) présente une forme sensiblement en forme de plaque pour fermer l'espace de réception (24) recouvert par la calotte sphérique creuse.

14. Agencement d'implant comprenant un implant (12) avec une première section d'implant (36) et une deuxième section d'implant (44) ainsi qu'un dispositif de conditionnement (10; 60; 70) selon l'une des revendications précédentes, où l'implant (12) est reçu dans un état de conditionnement du dispositif de conditionnement (10; 60; 70) dans l'espace de réception (24), où la première section d'implant (36) est reçue dans l'élément de réception (30) et l'élément de soutien (32) s'applique sur la deuxième section d'implant (44).

15. Agencement d'implant selon la revendication 14, **caractérisé en ce que** l'implant (12) est un composant tibial (14) d'une prothèse d'articulation du genou, qui comprend une tige tibiale (16) et un plateau tibial (18), où la tige tibiale (16) comprend ou forme la première section d'implant (36), et où le plateau tibial (18) comprend ou forme la deuxième section d'implant (44).
